# FASCICULE DE BREVET EUROPEEN

(11) **EP 1 219 615 B1**
(45) Date de publication et mention de la délivrance du brevet: **09.05.2007**
(21) Numéro de dépôt: 01403158.7
(22) Date de dépôt: 06.12.2001
(51) Int. Cl.: A61K 8/97, C07D 311/36, A23L 1/30

(54) **Procédé d'extraction de mélanges flavoniques et utilisation des mélanges ainsi obtenus en dermocosmétique, en alimentation et en pharmacie**
Verfahren zur Extraktion von Flavon-Gemischen, die dabei erhaltenen Gemische sowie deren Verwendung in Dermokosmetika, Ernährung und Pharmazeutika
Method for extracting flavon mixtures and use of mixtures so obtained in dermocosmetic, food and pharmacy

(30) Priorité: 13.12.2000 FR 0016240
(43) Date de publication de la demande: 03.07.2002
(73) Titulaire: LABORATOIRE THERAMEX, 98000 Monaco (MC)
(72) Inventeur: Lanquetin, Michel, Laguet, 06340 La Trinite (FR)
(74) Mandataire: Burtin, Jean-François

(56) Documents cités:
- EP-A- 0 795 553
- EP-A- 0 812 837
- US-A- 5 141 746
- US-A- 6 033 714

## Description

La présente invention se situe dans le domaine de la chimie et plus particulièrement dans celui de la chimie d'extraction de substances naturelles.

Elle a plus particulièrement pour objet un procédé d'extraction et d'enrichissement d'extraits végétaux en constituants biologiquement actifs.

Elle a précisément pour objet un nouveau procédé d'extraction conduisant à des produits riches en isoflavones à partir d'extraits ou de mélanges déjà concentrés en dérivés isoflavoniques et notamment en dérivés de la génistéine.

On connaît l'importance dans l'alimentation d'un apport important en isoflavones car la consommation significative qui en est faite en Extrême-Orient - et en particulier au Japon - par l'intermédiaire des produits du soja, a montré le rôle bénéfique pour la santé joué par ces composés, sans risque d'effet secondaire nocif.

La consommation de produits dérivés du soja est estimée à 60 à 100 mg d'isoflavones totales par jour alors que dans les pays occidentaux on consomme moins de 5 mg d'isoflavones chaque jour.

Ces pseudo-oestrogènes jouent un rôle bénéfique dans le maintien d'une action biologique similaire à celle des estrogènes naturels et surtout des estrogènes conjugués. Les isoflavones donnent de bons résultats dans le traitement des symptômes liés à la ménopause. Ils entraînent une diminution importante des troubles du climatère comme les bouffées de chaleur, l'amélioration de la dyspareunie et des vaginites. Une publication de Heart and Health Report (PR Newswire, 20 juin 2000), qui s'appuie sur 38 études a montré que le soja permet de réduire significativement le taux de cholestérol, et notamment le taux de LDL-cholestérol.

Cependant, l'administration de ces isoflavones ou phytoestrogènes s'est effectuée jusqu'ici par voie orale car les doses à administrer étaient relativement importantes. Compte tenu du fait que les laits de soja ou les jus de graine de soja contiennent peu d'isoflavones, en général de 20 à 70 mg de produit du soja pour 100 grammes, il est nécessaire, pour atteindre une dose efficace, d'utiliser ou d'administrer des quantités importantes de produits dérivés du soja.

Il existe donc un besoin certain en formes plus concentrées en isoflavones extraites du soja et présentant un pourcentage élevé en génistéine et en ses dérivés.
On connaissait déjà des dérivés O-malonylés en génistéine et en daidzéine présentant un rendement faible partant de 1 Kg de matière première et arrivant à 60 mg de produit final.
Une première approche a déjà été effectuée en concentrant les extraits de graines de soja par le procédé décrit dans le brevet européen 0.795.553 au nom de la société Archer Daniels Midland Co. au départ de mélasses de soja. On obtient ainsi des extraits qui contiennent cependant de faibles quantités d'isoflavones et principalement de la génistine (77 %) et un peu de daidzine (17 %).

Il est apparu encore nécessaire de chercher à obtenir des extraits plus concentrés présentant une teneur maximale en dérivés de génistine et surtout en composés O-malonylés de génistine, présents naturellement dans les préparations de soja.

Une deuxième approche a déjà été effectuée dans le brevet US 6 033 714 au nom de Archer Daniels Midland Co ayant pour objet un procédé de production d'isoflavones à partir de lait de soja en utilisant les différences de solubilité des isoflavones en fonction de la température. On obtient ainsi une fraction d'isoflavones d'une concentration comprise entre 55 et 70% en poids d'extrait sec mais dont la teneur en dérivés O-malonylés n'est pas mentionnée.

Le problème selon l'invention est donc de trouver un procédé qui permette d'obtenir sans dénaturation ou modification chimique des extraits de soja très riches en isoflavones sous forme O-malonylée et glycosylée dans lesquels le rapport génistéine/daidzéine total se trouve sensiblement identique à celui présent dans la nature.

Le problème de l'invention réside également dans le souci d'éliminer d'autres isoflavones peu actives ou complètement inactives comme principalement la glycitine et ses dérivés. De préférence, la teneur résiduelle en glycitine et ses dérivés sera inférieure à 4 % et d'une manière encore davantage préférée, inférieure à 1,5 %.

Le problème de l'invention réside encore dans la possibilité de réaliser des formes pharmaceutiques et notamment topiques à base de concentrés d'isoflavones suffisamment riches en principes actifs pour atteindre des concentrations actives sous un faible volume.

Un autre objet de l'invention consiste en la réalisation de préparations alimentaires, notamment de biscuits, de gaufrettes, de barres chocolatées et autres préparations solides ou fluides, contenant un concentré d'isoflavones de soja selon l'invention.
L'invention a encore pour objet l'obtention de concentrés d'extraits de soja dépourvus de glycitéine, renfermant des quantités élevées de dérivés O-malonylés d'isoflavones, supérieures à 10 % et pouvant atteindre 35 %, et contenant de préférence de 28 à 35 % d'isoflavones.

Ces concentrés trouvent un double usage d'une part en permettant d'obtenir des préparations riches en isoflavones et aussi en fournissant le moyen de renforcer la teneur en isoflavones dans des préparations moins concentrées pour garantir une préparation standardisée dont la teneur ne fluctue pas en fonction de celle résultant de la récolte de graines.

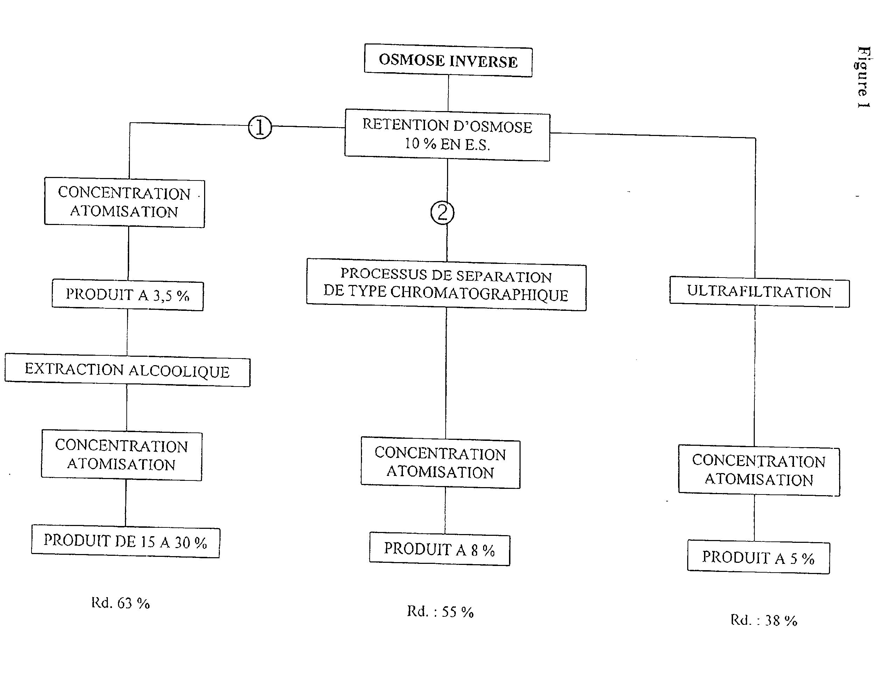

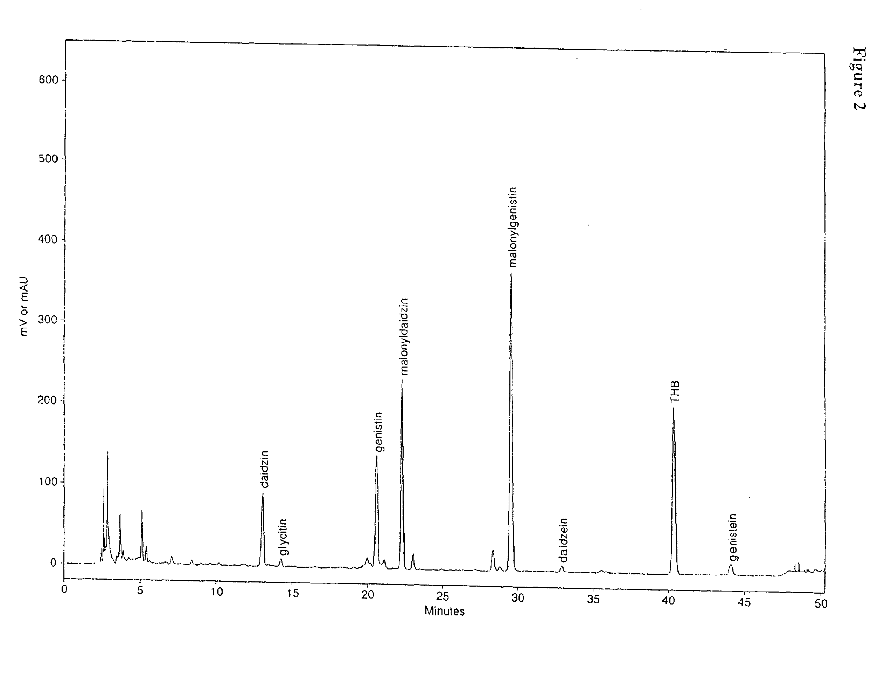

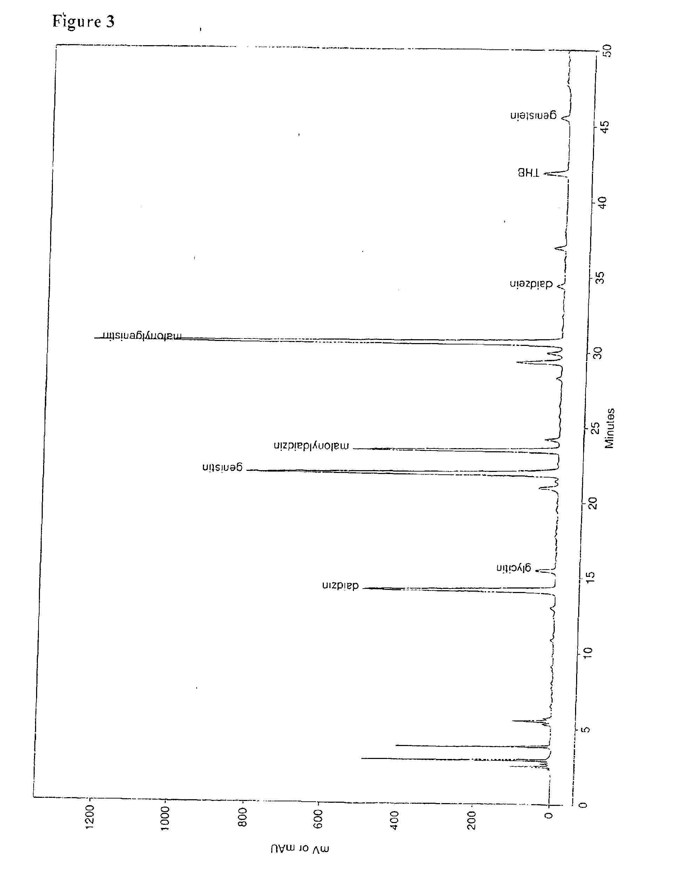

Ces problèmes ont trouvé une solution par l'utilisation d'un procédé nouveau de concentration d'un perméat de soja qui, par un procédé d'osmose inverse, a permis d'obtenir un rétentat d'osmose dont la concentration est voisine de 10 % en extrait sec. Ce rétentat constitue la matière première qui permet d'obtenir des mélanges pulvérulents possédant des concentrations en isoflavones et leurs dérivés, variant de 3,5 à environ 30 %, avec différents rendements de séparation. Le schéma de production annexé (figure 1) symbolise les différents processus de concentration.

Ces voies de production des différents produits de concentration d'isoflavones permettent d'obtenir des matières premières présentant un profil chromatographique d'une nature proche de celui observé dans la graine. Ces produits de concentration permettent, lorsqu'ils sont mélangés à des extraits moins riches, d'obtenir des concentrats dosés à 3,5-30 % sans dénaturation des produits obtenus.

Le procédé selon l'invention consiste essentiellement dans le fait qu'on traite des graines de soja préalablement dépelliculées en milieux aqueux pour obtenir un jus de soja, on soumet ce dernier à une première ultrafiltration sur membrane ayant un seuil de coupure supérieure à 10 000, pour produire un perméat de soja que l'on concentre par osmose inverse et obtient ainsi un concentré de soja riche en extrait sec, évapore ce concentré sous pression réduite pour recueillir une préparation de soja à forte teneur en isoflavones pouvant aller de 28 à 30 % environ d'extrait sec, épuise cette préparation par un solvant à bas point d'ébullition sans modification de profil, sans formation de dérivés acétylés non naturels puis atomise le résidu de cristallisation résultant, à basse température pour obtenir un mélange pulvérulent possédant une concentration en isoflavones supérieure à 30 %.
Le solvant à bas point d'ébullition est notamment un solvant hydroxylé, et plus particulièrement l'éthanol.

Dans un autre mode de réalisation du procédé selon l'invention le perméat de soja est concentré par osmose inverse pour obtenir un retentat d'osmose à 10 % de matières solides, séparation du type chromatographique et élution d'une fraction riche en isoflavones que l'on concentre sous pression réduite puis amenée à l'état sec par atomisation.
Par ce mode de réalisation, le rendement en isoflavones est de 55 % de la quantité théorique.

Le rétentat d'osmose peut aussi être soumis à une ultrafiltration, on recueille ensuite le rétentat que l'on concentre encore par atomisation et obtient une matière première présentant une teneur en isoflavones supérieure ou égale à 5 %. Le rendement de ce traitement est de l'ordre de 35 à 40%.

Les isoflavones obtenues par le procédé de l'invention se présentent essentiellement sous forme de dérivés O-malonylés et glycosylés de génistéine, très peu de glycitéine ou de glycitine.

En plus les préparations concentrées selon l'invention ne renferment pas, ou en quantité non détectable, de dérivés acétylés (acétyl daidzine, acétyl génistine, acétyl glycitine) ce qui indique que les opérations d'extraction ont été conduites à des températures suffisamment basses pour éviter une réaction de décarboxylation du dérivé malonylé et d'estérification en présence d'alcool.

Dans ces conditions, les proportions des isoflavones exprimées en aglycones d'isoflavones respectent celles trouvées dans la nature, soit environ 3/1/0-1 (génistéine / daidzéine / glycitéine).

Un avantage important du procédé selon l'invention provient du fait que les graines de soja sont au préalable dépelliculées. De cette façon, on élimine les germes qui rendent les concentrés non comestibles.

Le procédé selon l'invention fournit un concentré dans lequel les dérivés de la génistéine sont largement majoritaires alors que les procédés de l'art antérieur qui comportent des phases de recristallisation d'un extrait aqueux ou alcoolique fournissent des mélanges plus riches en daidzéine en raison des paramètres de solubilité de ce dernier composé. C'est ainsi qu'un produit commercial présentement sur le marché contient (en mg pour 100 g) :
5 595 mg de daidzine
1 809 mg de génistine
5 163 mg de glycitine
4 198 mg d'acétyldaidzine
1 670 mg d'acétylgénistine
3 354 mg d'acétylglycitine
(rapport génistéine / daidzéine / glycitéine / 0,39 / 1 / 0,97)

Ces chiffres permettent d'apprécier le degré beaucoup plus élevé de purification procuré par le procédé selon l'invention.

Les compositions pharmaceutiques, alimentaires, diététiques et cosmétiques contenant le concentré d'isoflavones selon l'invention, renferment des protéines et/ou des lipides, et/ou des glucides, et/ou des fibres alimentaires, et/ou des acides gras saturés, et/ou des acides gras polyinsaturés, et/ou des sels minéraux, et/ou des vitamines, et/ou des agents aromatisants, et/ou des édulcorants, et/ou des produits amylacés, et/ou des substances de charge minérales ou organiques, et/ou des agents de texture.

Dans le cas d'une barre chocolatée contenant des isoflavones, de 30 g, la valeur énergétique sera de l'ordre de 100 Kcal ou 800 à 1 000 Kj.

La teneur en acides gras polyinsaturés (acide linoléique, acide linolénique, EPA, DHA) peut être de 150 à 200 mg de DHA pour 100 g.

Dans une composition alimentaire selon l'invention, les teneurs en isoflavones s'échelonnent de 0,2 à 0,5 %, c'est à dire pour une barre chocolatée de 30 g une quantité s'échelonnant d'environ 60 à 150 mg par barre.

Parmi les éléments minéraux présents dans les compositions alimentaires selon l'invention, on pourra citer les dérivés du calcium, du phosphore, du magnésium, des métaux alcalins, du fer, du zinc, du cuivre, du manganèse, de l'iode et/ou du sélénium.

Parmi les vitamines contenues dans les compositions alimentaires selon l'invention, on pourra citer la vitamine A et ses précurseurs, la vitamine C, la vitamine B1, la vitamine B2, la vitamine PP, la vitamine B5, la vitamine B12, la vitamine B6, l'acide folique, le folinate de calcium et/ou la biotine.

L'invention concerne également les compositions cosmétiques à base de concentrés d'isoflavones selon l'invention, notamment sous forme de laits, de crèmes, de gels, de lotions, de bains, d'émulsions et similaires. On préparera ainsi des émulsions H/E anti-âge pour le contour des yeux, des gels anti-poches ou anti-cernes pour les yeux, des gels hydratants, des crèmes de jour protectrices, des crèmes adoucissantes ou des crèmes de soins pour les peaux desséchées.

L'invention a également pour objet les compositions pharmaceutiques renfermant à titre de principe actif les concentrés d'isoflavones en association ou en mélange avec un ou des excipients ou véhicules appropriés pour la voie digestive ou topique.

Les excipients ou diluants les plus utilisés sont le lactose, le carbonate de calcium, l'hydroxyde ou l'oxyde de magnésium, le phosphate tricalcique, le carbonate de magnésium, la cellulose microcristalline, la polyvinylpyrrolidone, la polyvinylpyrrolidone réticulée ou la polyvinylpolypyrrolidone.

On pourra ajouter à de telles préparations des agents liants comme l'éthylcellulose, des adjuvants de compression comme l'hydrotalcite ou une zéolite, des agents de glissement comme le stéarate de calcium ou le stéarate de magnésium ou des agents lubrifiants comme le talc et les esters de glycérol (stéarate, palmitate), ou des agents anti-électricité statique comme la silice colloïdale (Aerosil®).

On pourra également y incorporer des agents d'aromatisation synthétiques ou naturels (vanilline, éthylvanilline, maltol, furanéol, etc.), des protéines alimentaires comme la caséine ou ses hydrolysats ou des polysaccharides comme l'amidon ou les dextrines.

Les compositions à base d'extraits végétaux concentrés peuvent contenir de 5 à 200 mg d'isoflavones par prise unitaire. Les compositions seront administrées de 1 à 3 fois par jour et trouvent un emploi dans le traitement des troubles organiques liés à la ménopause. Elles s'adressent par exemple au traitement des bouffées de chaleur liées à la ménopause physiologique ou à la ménopause d'origine chirurgicale ou à la prévention des troubles liés aux phénomènes circulatoires, l'ostéoporose, et l'action anti-radicalaire entre autres.

Les compositions selon l'invention présentent une très bonne biodisponibilité après administration par voie orale, de comprimés notamment dosés à 37,5 mg et à la posologie de 2 comprimés / jour. Les essais de pharmacocinétiques ont été réalisés par administration unique sur un jour ou réitérés pendant 10 jours.
Les exemples suivants explicitent l'invention sans toutefois la limiter.

### EXEMPLE 1

### Préparation des poudres concentrées selon l'invention

On concentre un perméat de soja par osmose inverse à un débit de 400.000 litres/semaine pour obtenir un retentât d'osmose concentré 10 fois (débit 40.000 litres/semaine). Celui-ci est concentré par évaporation à pression réduite à raison de 15.000 litres/semaine pour obtenir un jus concentré à 12 % environ d'extrait sec et on élimine l'effluent.

Par évaporation supplémentaire à l'évaporateur rotatif, on recueille un concentré de jus de soja à 30 % d'extrait sec que l'on épuise à l'éthanol à basse température, évapore la phase éthanolique par atomisation à basse température et recueille une poudre jaune formée de 28 à 30 % d'isoflavones constituée principalement de génistéine et en second lieu de daidzéine.

Cette forme concentrée servira notamment à la réalisation de formes topiques pour la voie locale.

La poudre à 28.000 - 30.000 mg d'isoflavones / % g sert également à ajuster le titre de formes moins concentrées (6.000 à 8.000 mg / % g) à une valeur constante de 8.000 mg / % g.

Les diagrammes de chromatographie haute performance effectués sur des poudres à 3,5 % (diagramme 2) et à 30 % (diagramme 3) montrent que la concentration n'a pas eu d'incidence sur la composition qualitative du concentré.

### Avantage du procédé par concentration par osmose inverse du perméat de soja

La matière première de départ, le perméat de soja, est un produit très riche en eau (1 % d'extrait sec). La récupération des isoflavones contenues dans ce perméat n'est envisageable au stade industriel qu'à condition de réduire les volumes de liquide mis en oeuvre. La stabilité des isoflavones pouvant être altérée par un traitement thermique prolongé, une filtration dans des conditions de température n'excédant pas 50°C et permettant une élimination d'une partie de l'eau est adaptée. Cette filtration permettant la retenue totale des molécules de faible poids moléculaire que sont les isoflavones (PM de 254 Daltons à 532 Daltons) n'est possible que par un procédé, l'osmose inverse, utilisant des membranes de filtration de porosité très faible. Sous une pression de 20 à 40 bars, environ 90 % de l'eau contenue dans le perméat de soja est éliminé, amenant le rétentat obtenu à une concentration en extrait sec de 10 % environ. La proportion en isoflavones étant conservée, ce produit peut avantageusement être utilisé comme matière première pour l'extraction suivante.

### Compositions des poudres concentrées obtenues selon le procédé de l'EXEMPLE 1

### a - produit I

| **ISOFLAVONES** | **RESULTATS (mg/100 g de produit)** |
|---|---|
| Daidzine | 2 948,7 |
| Génistine | 3 013,5 |
| Glycitine | 296,1 |
| Malonyldaidzine | 6 770,0 |
| Malonylgénistine | 15 221,3 |
| Malonylglycitine | ND |
| Acétyldaidzine | ND |
| Acétylgénistine | ND |
| Acétylglycitine | ND |
| Daidzéine | 71,2 |
| Génistéine | 31,9 |
| Glycitéine | ND |
| TOTAL | 28352,7 |

### b - produit II

| **ISOFLAVONES** | **RESULTATS (mg/100 g de produit)** |
|---|---|
| Daidzine | 2 749,6 |
| Génistine | 2 517,2 |
| Glycitine | 284,2 |
| Malonyldaidzine | 8 245,6 |
| Malonylgénistine | 17 121,0 |
| Malonylglycitine | ND |
| Acétyldaidzine | ND |
| Acétylgénistine | ND |
| Acétylglycitine | ND |
| Daidzéine | 56,3 |
| Génistéine | 22,6 |
| Glycitéine | ND |
| TOTAL | 31 006,5 |

| | |
|---|---|
| ND = non déterminable | |

### EXEMPLE II

### Formulations cosmétiques : exemples de réalisation de compositions

| **1 - Crème de jour protectrice** | |
|---|---|
| Isoflavones de soja | 0,1 à 0,5 % |
| Alphahydroxyacides | 2 - 4 % |
| Vitamine A | 0,25 - 1 % |
| Filtre solaire organique | 3 - 6 % |
| Agent anti-radicalaire | 2 - 5 % |
| Agent hydratant | 2 - 3 % |
| Emollient | 15 - 20 % |
| Agent émulsionnant H/E | 5 - 10 % |
| Agent gélifiant | 1 - 2 % |
| Conservateur | 0,1 - 0,5 % |
| Parfum | 0,1 - 0,3 % |
| Eau purifiée qsp 100 | 69,45 - 47,7 % |
| | |

| **2 - Emulsion H/E anti-âge** | |
|---|---|
| Isoflavones de soja | 0,1 - 0,5 % |
| Alphahydroxyacides | 2,5 - 10 % |
| Vitamine A | 0,25 - 1 % |
| Agent hydratant | 2 - 3 % |
| Emollient | 15 - 20 % |
| Agent émulsionnant H/E | 5 - 10 % |
| Agent gélifiant | 1 - 2 % |
| Conservateur | 0,1 - 0,5% |
| Parfum | 0,1 - 0,3 % |
| Eau purifiée qsp 100 | 73,95 - 52,7 % |
| | |

| **3 - Lait H/E anti-âge pour le corps** | |
|---|---|
| Isoflavones de soja | 0,1 - 0,5 % |
| Alphahydroxyacides | 2,5 - 10 % |
| Vitamine A | 0,25 - 1 % |
| Agent hydratant | 2 - 3 % |
| Emollient | 15 - 20 % |
| Agent émulsionnant H/E | 2 - 4 % |
| Agent gélifiant | 1 - 2 % |
| Conservateur | 0,1 - 0,5 % |
| Parfum | 0,1 - 0,3 % |
| Eau purifiée qsp 100 | 74,35 - 58,7 % |
| | |

| **4 - Emulsion H/E anti-âge contour des yeux** | |
|---|---|
| Isoflavones de soja | 0,1 - 0,5 % |
| Ester d'alpha hydroxyacide | 2,5 - 5,0 % |
| Vitamine A | 0,25 - 1 % |
| Agent hydratant | 2 - 3 % |
| Agent filmogène | 1 - 2 % |
| Emollient | 15 - 20% |
| Agent émulsionnant | 5 - 10 % |
| Agent gélifiant | 1 - 2 % |
| Conservateur | 0,1 - 0,5 % |
| Parfum | 0,1 - 0,3 % |
| Eau purifiée qsp 100 | 72,95 - 55,7% |
| | |

| **5 - Gel anti-poches / anti-cerne** | |
|---|---|
| Isoflavones de soja | 0,1 - 0,5 % |
| Extrait de végétaux | 5 - 10 % |
| Agent hydratant | 2 - 3 % |
| Emollient | 3 - 5% |
| Agent gélifiant | 2 - 4 % |
| Conservateur | 0,2 - 0,3 % |
| Eau purifiée qsp 100 | 87,7 - 76,7 % |
| | |

| **6 - Gel hydroalcoolique** | |
|---|---|
| Isoflavones de soja | 0,3 - 2 % |
| Agent relipidant | 1 - 5 % |
| Agent gélifiant | 2 - 3 % |
| EtOH 95° | 30 - 80 % |
| Eau purifiée qsp 100 | 66,5 - 9,2 % |
| Conservateur | 0,1 - 0,5 % |
| Parfum | 0,1 - 0,3 % |

### EXEMPLE III

### Formulations diététiques

| **1 - Barres au chocolat (30 ou 60 g environ)** | | | |
|---|---|---|---|
| | | en g/100 g | pour 60 g |
| Protéines | | 23,01 | 13,8 |
| (NT 6,25) | | | |
| | de soja | 11,35 | |
| | de lait | 11,66 | |
| Lipides | | 13,1 | |
| Glucides | | 37,4 | |
| (dont mono et di-alimentaires) | | 32 | |
| Fibres alimentaires dont FOS | | 13 | |
| Valeur énergétique | | 368 kcal | |
| | | 1537 kj | |
| AG saturés | | 3,12 | 1,9 |
| AG mono insaturés | | 3,07 | 1,8 |
| AG polyinsaturés | | 6,88 | 4,1 |
| | Ac.linoléique | 3,4 | 2,1 |
| | Ac.linolénique | 0,50 | 0,3 |
| | EPA | 113 (en mg/100 g) | 68 mg |
| | DHA | 166 (en mg/100 g) | 100 mg |
| | | | |
| Isoflavones à 25 % de principe actif | | 99 mg | 89 |
| Minéraux | | (mg/100g) | mg (0,2 à 0,5 %) en mg |
| | Calcium | 404 à 340 | 242 à 35 |
| | Phosphore | 287 400 | 172 31 |
| | Magnésium | 83 75 | 49,9 33 |
| | Sodium | 267 300 | 160 28 |
| | Potassium | 1552 810 | 931 30 |
| | Fer | 10,3 8 | 6,2 39 |
| | Zinc | 8,5 5 | 5,1 54 |
| | Cuivre | 0,576 0,6 | 0,3 31 |
| | Manganèse | 0.387 0,5 | 0,2 23 |

| | | | Pour 60 g |
|---|---|---|---|
| | Iode | 70 µg | 32 à 42 |
| | Sélénium | 31 µg | 18,7 à 34 |
| | | | |

| **Vitamines** | | | |
|---|---|---|---|
| | A(ER) | 367 µg | 31 à 220 |
| | E | 5,0 mg | 3,0 30 |
| | D3 | 2,7 µg | 1,6 32 |
| | C | 35,5 mg | 21,3 47 |
| | B1 | 0,6 mg | 0,4 33 |
| | B2 | 0,8 mg | 0,5 31 |
| | B6 | 1,8 mg | 0,6 43 |
| | PP | 9,7 mg | 5,8 32 |
| | B5 | 1,85 mg | 1,1 37 |
| | Acide Folique | 108 µg | 64,8 32 |
| | Biotine | 85 µg | 51,0 34 |
| | | | |

| **2 - Biscuits vitaminés** | | | |
|---|---|---|---|
| Protéines végétales | | 75 g | |
| Caséine | | 254 g | |
| Petit-lait | | 47 g | |
| Isoflavones à 25 % de principes actifs | | 14 g | |
| Lécithine de soja | | 60 g | |
| Beurre doux hydrogéné | | 55 g | |
| Poudre de lait | | 81 g | |
| Poudre d'oeuf | | 12 g | |
| Sucre vanillé à 1 % | | 117 g | |
| Concentré de jus d'orange | | 70 g | |
| Polymère acrylique | | 21 g | |
| Farine de céréales | | 1 300 g | |
| Amidon de blé (pour 100 biscuits de 30 g) | | 980 g | |
| | | | |

| **3 - Biscuits Dynamisants** | | | |
|---|---|---|---|
| Acide Ascorbique | | 2.500 mg | |
| Farine de blé | | 160 g | |
| Lécithine de soja | | 12 g | |
| Matières grasses végétales | | 7,5 g | |
| Poudre d'isoflavones à 25 % de principes actifs | | 5 g | |
| Poudre de lait | | 4,1 g | |
| Poudre d'oeuf | | 2,75 g | |
| Concentré de sirop de café | | 41 g | |
| Carbonate de calcium | | 8 g | |
| Phosphate de magnésium | | 6 g | |
| Oxyde de zinc | | 0,5 g | |
| Hydroxyéthylcellulose (Pour des biscuits pesant 10 g environ) | | 10 g | |
| | | | |

| **4- Comprimés à sucer** | | | |
|---|---|---|---|
| Poudre d'isoflavones à 30 % de principe actif | | 3,5 g | |
| Acétate de tocophérol | | 0,5 g | |
| Acide silicique | | 20 g | |
| Cellulose micro cristalline | | 10 g | |
| Stéarate de magnésium | | 30 g | |
| Lactose | | 60 g | |
| Poudre de chocolat | | 10 g | |
| Xylitol | | 10 g | |

Le mélange a été comprimé en tablettes ayant un diamètre de 17 mm et un poids moyen de 150 mg.

| **5 - Granulé chocolaté** | |
|---|---|
| Silice colloïdale | 20,5 g |
| Poudre d'isoflavones à 30 % | 0,5 g |
| Amidon de maïs | 79 g |
| Cellulose microcristalline | 14 g |
| Sucre 10 g | |

100 ml d'une solution à 10 % d'hydroxy propyl cellulose dans l'éthanol
Le mélange a été broyé, extrudé en filaments et séché pour former des granulés.

### EXEMPLE IV

### Formulations pharmaceutiques

| **1 - Comprimés à 75,0 mg d'isoflavones** | |
|---|---|
| - Extrait de graines de soja titré à 16 % d'isoflavones | 235,00 g |
| - Amidon modifié (Sépislab) | 231,00 g |
| - Compritol E | 6,00 g |
| - Aérosil 200 (silice colloïdale) | 1,25 g |
| - Stéarate de magnésium | 3,00 g |
| - Lactose | 150,00 g |
| Poids total pour 1000 comprimés dosés à 37,5 mg | 626,25 g |

| | |
|---|---|
| 2 cps/jour = 75 mg | |

| **2 - Gélules** | |
|---|---|
| - Extrait de graines de soja titré à 16 % d'isoflavones | 234,375 g |
| - Lactose | 100,000 g |
| - Aérosil 200 | 6,125 g |
| - Stéarate de magnésium | 10,500 g |
| Poids pour 1000 gélules | 351,000 g |

| | |
|---|---|
| Chaque gélule contient 37,50 mg d'isoflavones. | |

| **3 - Capsules molles** | | |
|---|---|---|
| a) | Extrait de graines de soja titré à 28 % d'isoflavones | 468,75 g |
| | Lécithine | 10,25 g |
| | Huile de soja | 211,00 g |
| | | 690,00 g |

| | | |
|---|---|---|
| pour 1000 capsules 14 N oblongues Chaque capsule contient 125 mg d'isoflavones. | | |

| **4 - Capsules** | |
|---|---|
| Extrait de graines de soja titré à 28 % d'isoflavones | 450 g |
| Lécithine | 10,125 g |
| Huile de soja hydrogénée | 100,500 g |
| | 560,625 g |

| | |
|---|---|
| pour 1000 capsules 6 N ovales Chaque capsule contient 125 mg d'isoflavones. | |

| **5 - Comprimés** | |
|---|---|
| - Extrait de graines de soja titré à 28 % d'isoflavones | 22,500 kg |
| - Sepistab ST 200 | 6,800 kg |
| - Béhénate de glycérol (Compritol E) | 0,300 kg |
| - Silice colloïdale | 0,250 kg |
| -Stéarate de magnésium | 0,350 kg |
| | 30,200 kg |

| | |
|---|---|
| pour 48000 comprimés terminés à 0,628 g Chaque comprimé contient 125 mg d'isoflavones. | |

| **6 - Comprimés dragéifiés** | |
|---|---|
| - Extrait de graines de soja titré à 28 % d'isoflavones | 22,682 kg |
| - Sepistab ST 200 | 6,835 kg |
| - Béhénate de glycérol | 0,290 kg |
| - Silice colloïdale | 0,048 kg |
| - Stéarate de magnésium | 0,145 kg |
| - Sepifilm LP010 | 0,900 kg |
| - Sepifilm SC 758 blanc | 5,850 kg |
| - Talc | 2,250 kg |
| - Sepisperse rose | 1,200 kg |
| - Cire de carnauba | 0,009 kg |
| | 40,209 kg |

| | |
|---|---|
| pour 48000 comprimés dragéifiés roses de 0,830 g | |

| **7 - Comprimés dragéifiés** | |
|---|---|
| - Extrait de graines de soja titré à 28 % d'isoflavones | 22,682 kg |
| - Sepistab ST 200 | 5,141 kg |
| - Levure sélénisée | 1,694 kg |
| - Béhénate de glycérol | 0,290 kg |
| - Silice colloïdale | 0,048 kg |
| - Stéarate de magnésium | 0,145 kg |
| - Sepifilm LP 010 | 0,900 kg |
| - Sepifilm SC 758 | 5,850 kg |
| - Talc | 2,250 kg |
| - Sepisperse AS 3174 vert | 1,200 kg |
| - Cire de carnauba | 0,009 kg |
| | 40,209 kg |

| | |
|---|---|
| pour 48000 comprimés dragéifiés verts, terminés au poids de 0,835 g dosés à 125 mg d'isoflavones. | |

| **8 - Comprimés dragéifiés** | |
|---|---|
| - Extrait de graines de soja titré à 28 % d'isoflavones | 22,682 kg |
| - Sepistab ST 200 | 4,538 kg |
| - Citrate de zinc | 2,297 kg |
| - Béhénate de glycérol | 0,290 kg |
| - Silice colloïdale | 0,048 kg |
| - Stéarate de magnésium | 0,145 kg |
| - Sepifilm LP 010 | 0,900 kg |
| - Sepifilm SC 758 | 5,850 kg |
| - Talc | 2,250 kg |
| - Sepisperse orange | 1,200 kg |
| - Cire de carnauba | 0,009 kg |
| | 40,200 kg |

| | |
|---|---|
| pour 48000 comprimés dragéifiés orange terminés à 0,835 g et dosés à 125 mg d'isoflavones. | |

| **9 - Granulés** | |
|---|---|
| - Extrait de graines de soja titré à 30 % d'isoflavones | 22,682 kg |
| - Lactose | 10,282 kg |
| - Cellulose microcristalline | 18,036 kg |
| - Polyvinylpyrrolidone K 25 | 9,000 kg |
| | 60,000 kg |

| | |
|---|---|
| Après granulation humide et séchage, obtention de 56 kg de granulé soit environ 130 mg d'isoflavones pour 2,5 g de granulé | |

| **10 - Comprimés enrobés** | |
|---|---|
| - Extrait de graines de soja titré à 28 % d'isoflavones | 22,682 kg |
| - Lactose | 6,835 kg |
| - Silice colloïdale | 0,053 kg |
| - Stéarate de magnésium | 0,145 kg |
| - Palmitostéarate de glycérol | 0,300 kg |
| - Eudragit pH résistant | 0,900 kg |
| - Sépisperse vert | 1,200 kg |
| | |
| - Cire de carnauba | 0,085 kg |
| | 32,200 kg |

| | |
|---|---|
| pour 48000 comprimés pelliculés gastrorésistants verts terminés à 0,670 g et dosés à 125 mg d'isoflavones | |

| **11 - Comprimés dragéifiés** | |
|---|---|
| - Extrait de graines de soja titré à 28 % d'isoflavones | 22,862 kg |
| - Sepistab ST 200 | 6,259 kg |
| - Fumarate de fer | 0,576 kg |
| - Béhénate de glycérol | 0,290 kg |
| - Silice colloïdale | 0,048 kg |
| - Stéarate de magnésium | 0,145 kg |
| - Sepifilm LP 010 | 0,900 kg |
| - Sepifilm SC 758 blanc | 5,850 kg |
| - Talc | 2,250 kg |
| - Cire de carnauba | 0,020 kg |
| | 39,200 kg |

| | |
|---|---|
| pour 48000 comprimés dragéifiés blancs terminés à 0,816 g et dosés à 125 mg d'isoflavones | |

| **12 - Comprimés dragéifiés** | |
|---|---|
| - Extrait de graines de soja titré à 28 % d'isoflavones | 22,862 kg |
| - Sepistab ST 200 | 6,259 kg |
| - Carbomère carboxyvinylique | 6,619 kg |
| - Silice colloïdale | 0,250 kg |
| - Sepifilm LP 010 | 0,900 kg |
| - Sepifilm SC 758 blanc | 8,100 kg |
| - Cire de carnauba | 0,010 kg |
| | 45,000 kg |

| | |
|---|---|
| pour 73 000 comprimés matriciels terminés à 0,615 g et dosés à 125 mg d'isoflavones | |

Pour les dragées, le procédé de fabrication est le suivant :
1. Compression, Séchage des noyaux par le Sepifilm LP 010
2. Montage Sepifilm SC + Talc
3. Enrobage par la cire de Carnauba

## Revendications

1. Concentrés de soja riches en isoflavones sous forme O-malonylée sans dénaturation ni modification chimique, de la forme native dans lequel les extraits obtenus se caractérisent par une teneur en isoflavones supérieur à 10% en poids, par un rapport génistéine/daidzéine sensiblement identique à celui présent dans la nature et par une absence quasi complète de dérivés de glycitéine et possèdent une teneur en dérivés O-malonylés d'isoflavones s'échelonnant de 10% à 35% en poids.

2. Concentrés de soja riches en isoflavones sous forme O-malonylée sans dénaturation ni modification chimique de la forme native selon la revendication précédente dans lequel la teneur en glycitine et ses dérivés dans les concentrés est de 4% ou inférieure à 4 %.

3. Concentrés de soja riches en isoflavones sous forme O-malonylée sans dénaturation ni modification chimique de la forme native selon la revendication 1 ou la revendication 2 dans lequel la teneur en glycitine et ses dérivés dans les concentrés est inférieure à 1,5 %.

4. Produits de concentrés de soja riches en isoflavones sous forme O-malonylée sans dénaturation ni modification chimique de la forme native selon les revendications précédentes **caractérisés en ce qu'**ils présentent un rapport génistine/daidzine/glycitine voisin de 3/1/0.

5. Procédé pour obtenir des extraits de soja concentrés en isoflavones selon l'une des revendications précédentes sans dénaturation ni modification chimique **caractérisé en ce qu'**on traite les graines de soja préalablement dépelliculées en milieux aqueux pour obtenir un jus qu'on soumet à une première ultrafiltration pour produire un perméat de soja, qu'on concentre celui-ci par osmose inverse et obtient un concentré de soja riche en extrait sec, évapore ce concentré sous pression réduite pour isoler une préparation à forte teneur en isoflavones, épuise cette préparation par un solvant à bas point d'ébullition sans modification de profil, puis atomise le résidu résultant à basse température pour obtenir un mélange pulvérulent possédant une concentration en O-malonyl isoflavones qui s'échelonne de 10 à 35 %, en poids.

6. Procédé selon la revendication 5, dans lequel l'ultrafiltration préliminaire est effectuée sur une membrane ayant un seuil de coupure supérieur à 10.000.

7. Procédé selon la revendication 5, dans lequel la concentration par osmose inverse est effectuée dans une cellule à membrane de filtration, de porosité très faible.

8. Procédé selon la revendication 5, dans lequel l'évaporation du concentré s'effectue sous pression réduite.

9. Procédé selon la revendication 5, dans lequel on utilise comme moyen d'évaporation un évaporateur rotatif.

10. Procédé selon la revendication 5, dans lequel l'extraction par solvant à bas point d'ébullition, du concentré de soja, est effectuée avec un solvant hydroxylé.

11. Procédé selon la revendication 5, dans lequel le solvant hydroxylé est l'éthanol.

12. Procédé selon la revendication 5, dans lequel le produit d'extraction éthanolique est amené à sec par atomisation sous pression réduite.

13. Procédé selon la revendication 5, dans lequel le produit d'atomisation est une poudre titrant de 280 à 350 mg/g d'isoflavones, notamment sous forme O-malonylée.

14. Concentrés d'isoflavones extraits de graines de soja titrant de 100 à 350 mg/g d'isoflavones sous forme O-malonylée, obtenus par le procédé de l'une des revendications 5 à 13.

15. Formulations topiques utilisables en cosmétique et en dermatologie, dans lesquelles l'ingrédient actif est un concentré d'isoflavones selon la revendication 1.

16. Formulations topiques, notamment crèmes, laits, lotions, gels, bains ou émulsions, dans lesquelles l'ingrédient actif est un concentré d'isoflavones selon la revendication 1.

17. Compositions alimentaires ou diététiques, dans lesquelles l'ingrédient principal est un concentré d'isoflavones selon la revendication 1.

18. Compositions alimentaires selon la revendication 17, **caractérisées en ce qu'**elles renferment en outre des protéines, des lipides, des glucides, des fibres alimentaires, des acides gras, des sels minéraux, des vitamines, des substances aromatisantes, des édulcorants, des produits amylacés, des substances de charge minérales ou organique et des agents de texture.

19. Compositions pharmaceutiques destinées à la voie orale ou à la voie topique **caractérisées en ce qu'**elles renferment, à titre d'ingrédient actif, un concentré d'isoflavones selon la revendication 1.

## Claims

1. O-malonyl isoflavone-rich soy concentrates without denaturation or chemical modification of the native form, in which the obtained extracts are **characterized by** an isoflavone content of above 10% w/w, by a ratio genistein/daidzein about identical to the natural ratio and by the absence of glycitein derivatives, and have a O-malonyl isoflavone content between 10% and 35% w/w.

2. O-malonyl isoflavone-rich soy concentrates without denaturation or chemical modification of the native form according to claim 1, in which the glycitein and glycitein derivative content is of 4% or less.

3. O-malonyl isoflavone-rich soy concentrates without denaturation or chemical modification of the native form according to claim 1 or claim 2, in which the glycitein and glycitein derivative content is less than 1.5%.

4. Products from O-malonyl isoflavone-rich soy concentrates without denaturation or chemical modification of the native form according to the preceding claims, **characterized in that** their ratio genistein/daidzein/glycitein is around 3/1/0.

5. Process to obtain isoflavone-rich soy concentrate extracts according to one of the preceding claims, without denaturation or chemical modification of the native form, **characterized in that** the peeled soy beans are treated in aqueous medium to give a juice, which is then submitted to a first ultrafiltration to give a soy ultrafiltrate, which is concentrated by reverse osmosis to give a dry extractrich soy concentrate, which is then evaporated under vacuum to isolate an isoflavone-rich preparation ; this preparation is filtered with suction with a low boiling point solvent without profile modification ; the residue is atomized at low temperature to obtain a pulverulent mixture whose O-malonyl isoflavone concentration is comprised between 10 and 35% w/w.

6. Process according to claim 5, in which the preliminary ultrafiltration is done on a membrane with a molecular weight cutoff above 10,000.

7. Process according to claim 5, in which the reverse osmosis concentration is done in a low-porosity filtration membrane cell.

8. Process according to claim 5, in which the concentrate is evaporated under vacuum.

9. Process according to claim 5, in which the evaporation apparatus is a rotary evaporator.

10. Process according to claim 5, in which the extraction of the soy concentrate with a low boiling point solvent is done with a hydroxylated solvent.

11. Process according to claim 5, in which the hydroxylated solvent is ethanol.

12. Process according to claim 5, in which the ethanolic extractive is dried by low-pressure atomisation.

13. Process according to claim 5, in which the atomisation product is a powder containing from 280 to 350 mg/g of isoflavones, especially O-malonyl isoflavones.

14. Isoflavone concentrates extracted from soy beans, containing from 100 to 350 mg/g of O-malonyl isoflavones, obtained by a process according to one of the claims 5 to 13.

15. Topical formulations usable for cosmetic or dermatological purposes, in which the active ingredient is an isoflavone concentrate according to claim 1.

16. Topical formulations, especially creams, milks, lotions, gels, baths or emulsions, in which the active ingredient is an isoflavone concentrate according to claim 1.

17. Alimentary or dietary compositions, in which the active ingredient is an isoflavone concentrate according to claim 1.

18. Alimentary compositions according to claim 17, **characterized in that** they also contain proteins, lipids, carbohydrates, dietary fibres, fatty acids, mineral salts, vitamins, flavouring agents, sweeteners, starch compounds, organic or mineral charge substances and texturizing agents.

19. Pharmaceutical compositions for oral or topical administration, **characterized in that** they contain, as the active ingredient, an isoflavone concentrate according to claim 1.

## Patentansprüche

1. Konzentrate von Soja die an Isoflavonen reich sind, in der Form von O-malonyl Derivaten, ohne Denaturation noch chemische Modifiezierung der ursprunglische Form, worin die hergestellte Extrakten **gekennzeichnet sind durch** eine Gehalt an Isoflavonen höher als 10 % bei Gewicht, **durch** ein Verhältnis Genistein / Daidzein der ungefähr identisch ist als der in der Natur anwesend ist und **durch** ein quasi vollständige Mangel an Glycitin Derivaten und die **gekennzeichnet sind durch** einen Gehalt an O-malonylierte Derivaten von Isoflavonen, der sich von 10 bis 35 % bei Gewicht erstreckt.

2. Konzentrate von Soja die an Isoflavonen reich sind, unter der Form von O-malonylierte, ohne Denaturierung noch chemische Veränderung der unsprunglischen Form, nach der vorstehende Anspruch, worin der Gehalt an Glycitin und seiner Derivaten in den Konzentraten, von 4 % oder niedriger als 4 % ist.

3. Konzentrate von Soja an Isoflavonen unter O-malonylierte Form, ohne Denaturie rung noch chemische Veränderung der ursprunglischen Form, nach Anspruch 1 oder Anspruch 2, worin der Gehalt an Glycitin und seiner Derivaten, niedriger als 1,5 % ist.

4. Produkten von Konzentrate von Soja, die reich an Isoflavon in der Form von O-malonylierten Derivaten sind, ohne Denaturierung oder chemische Veränderung der ursprunglischen Form, nach der vorstehenden Ansprüche, **dadurch** gekennzeichet sind, daß sie ein Verhältnis Genistein / Daidzein / Glycitin in der Nähe von 3/1/0 vorstellen.

5. Vefahren zur Herstellung von an Isoflavonen Konzentrierten Soja Extrakten, nach einer der vorstehenden Ansprüche, ohne Denaturierung noch chemische Veränderung, **dadurch** gekennzeichet ist daß man die Samen von Soja die vorher entuberzügt werden, in wässrigen Medium behandelt um eine Saft zu erzeugen, der an einer ersten Extraktion unterwirft wird, um einen Permeat von Soja zu erzeugen, anreicht dieses durch Reverse- Osmosis und einen Konzentrat von Soja der an trocknem Extrakt reich ist, ergibt, diesen Konzentrat unter verminderte Drück abdämpft eine Hochprozentige Bereitung an Isoflavonen abzutrennen, erschöpft diese Bereitung durch eine niedrigsiedende Lösungsmittel ohne Veränderung des Ansicht, danach man den resultierende Rückstand am niedrige Temperatur atomisiert um eine pulvrige Mischung zuherstellen die einen Gehalt an O-Malonyl isoflavonen enthält, der von 10 bis 35% bei Gewicht sich erstreckt.

6. Verfahren nach Anspruch 5, worin die vorläufige Ultrafiltration über einen Membran mit eine Schneidungsgrenze höher als 10.000, überfuhrt.

7. Verfahren nach Anspruch 5, worin die Konzentrierung nach Reverse -Osmosis in einer Zell mit Filtrierungs- Membran die eine sehr schwache Porosität ausweist, ausführt wird.

8. Verfahren nach Anspruch 5, worin die Verdampfung des Konzentrats unter verminderte Druck ausgeführt wird.

9. Verfahren nach Anspruch 5, worin als Verdämpfungsmittel ein rotierende Verdampfer gebraucht wird.

10. Verfahren nach Ausprunch 5, worin die Extraktion mit einem niedrigsiedende Lösungsmittel, des Konzentrats von Soja, mit einen hydroxylierten Lösungsmittel ausgeführt wird.

11. Verfahren nach Anspruch 5, worin das hydroxyliertes Lösungsmittel Ethanol ist.

12. Verfahren nach Anspruch 5, worin man das Produkt von ethanolischen Extraktion zum Trockenen durch Atomisation unter verminderten Drück bringt.

13. Verfahren nach Ansprunch 5, worin man das Atomisationsprodukt, eine Pulver die von 250 bis 350 mg/g von Isoflavonen, nämlich unter der O-Malonyl Form, titriert.

14. Konzentraten die von Isoflavonen aus Soja Samen extraktierte sind, von 150 bis 350 mg von Isoflavone titrierend, die unter der O-Malonyl Form sind, die nach dem Verfahren von einer der Ansprüche 5bis 13 hergestellt sind.

15. Topische Bereitungen die in Kosmetik und in Dermatologie brauchbar sind, worin der Wirkstoff eine Konzentrat von Isoflavonen nach Anspruch 1, ist.

16. Topische Bereitungen, vor allem Cremes, Milch, Lotion, Bade, oder Emulgierungen, worin der Wirkstoff eine Konzentrat von Isoflavonen nach Anspruch 1, ist.

17. Ernährungsmittel oder diätetische Zusammensetzungen worin der Hauptwirkstoff eine Konzentrat von Isoflavonen nach Anspruch 1, ist.

18. Ernährungs Zusammensetzungen nach Anspruch 17, **dadurch gekennzeichnet sind daß** sie außerdem, Protein, Lipiden, Gluziden, Ernährungsfiber, Fettsäure, mineral Salzen, Vitaminen, Riechstoffe, Süßstoffe, starkenhaltige Substanzen, mineralischen oder organischen Füllmaterial und Texturmittel enthalten.

19. Pharmazeutische für die orale Abreichung oder die topische Applikation vorgesehen, Zubereitungen **dadurch gekennzeichnet sind**, das sie als Wirkstoff, eine Konzentrat von Isoflavonen nach der Anspruch 1, enthalten.
